# EUROPÄISCHE PATENTSCHRIFT

(11) **EP 2 373 637 B1**
(45) Veröffentlichungstag und Bekanntmachung des Hinweises auf die Patenterteilung: **22.05.2013**
(21) Anmeldenummer: 09764502.2
(22) Anmeldetag: 02.12.2009
(51) Int. Cl.: C07D 309/30, B01J 23/80, B01J 23/89

(54) **VERFAHREN ZUR HERSTELLUNG VON DELTA-VALEROLACTON IN DER GASPHASE**
PROCESS OF PREPARATION IN THE GAS PHASE OF DELTA-VALEROLACTONE
PROCÉDÉ DE PRÉPARATION EN PHASE GAZEUSE DE LA DELTA VALEROLACTONE

(30) Priorität: 05.12.2008 EP 08170862
(43) Veröffentlichungstag der Anmeldung: 12.10.2011
(73) Patentinhaber: BASF SE, 67056 Ludwigshafen (DE)
(72) Erfinder: PINKOS, Rolf, 67098 Bad Dürkheim (DE); BAUDUIN, Christophe, 68723 Plankstadt (DE); PAUL, Axel, 68623 Lampertheim (DE); FRITZ, Gerhard, 67125 Dannstadt-Schauernheim (DE); WAGNER, Hans, 67269 Grünstadt (DE)
(86) Internationale Anmeldenummer: PCT/EP2009/066186
(87) Internationale Veröffentlichungsnummer: WO 2010/063742

(56) Entgegenhaltungen:
- EP-A1- 1 214 972
- WO-A1-90/14344
- CN-A- 101 157 677
- GB-A- 583 344
- JP-A- 4 261 167
- JP-A- 2004 331 626

## Beschreibung

Das Verfahren betrifft ein Verfahren zur Herstellung von delta-Valerolacton (VLO) in der Gasphase durch katalytische Dehydrierung an mindestens zwei verschiedenen Katalysatoren.

VLO ist ein gesuchter Ausgangsstoff zur Herstellung cyclischer Lactame, als Intermediat für Polylactone und zur Herstellung für Pharmazeutika bzw. für Pflanzenschutzmittel.

In EP-A 1214972 wird die Herstellung von gamma-Butyrolactom in der Gasphase aus 1,4-Butandiol beschrieben. Allgemein findet sich in dieser Offenbarung auch die Lehre, dass man aus dem entsprechenden Pentandiol VLO herstellen kann. Die Verwendung von wenigstens zwei Katalysatoren für diese Umsetzung, die in Schichten angeordnet sind, ist jedoch nicht offenbart. Versuche und Versuchsbedingungen zur gezielten Herstellung von VLO aus 1,5-Pentandiol sind aus dem Stand der Technik nur wenig bekannt.

In JP 2004331626 ist in einem Beispiel erwähnt, dass sich VLO an einem Cuoxid/Silika-Katalysator, der bei 260°C gehalten wurde, in der Gasphase herstellen lässt. Es wurde ein Reaktionsaustrag erhalten, der 85 Gew.-% VLO enthielt. Der Einsatz zweier verschiedener Katalysatoren zur Herstellung von VLO wird hier nicht beschrieben.

Die Umsetzung eines Diols zum entsprechenden Lacton unter Wasserstoffabspaltung und Sauerstoffausschluss ist eine endotherme Reaktion. Zur Erzielung hoher Umsätze muss dem System daher Wärme zugeführt werden. Dabei dürfen die Wegstrecken zwischen den heißen Zonen nicht zu weit auseinander liegen, sonst kommt es zu einer unerwünschten Abkühlung des Gasstroms und damit zu geringem Umsatz. Ferner kann das hochsiedende Diol auf dem Katalysator auskondensieren, was generell zu kurzen Lauf- oder Standzeit des Katalysators, z.B. durch Verkokung, führt. Technisch geschieht die Zufuhr von Wärme mittels eines Rohrbündelreaktors, bei dem katalysatorführende Rohre mit Durchmessern von z.B. 3 cm durch ein Wärmemedium wie Salzbad oder Dampf zur Wärmeeinbringung verlaufen. Rohrbündelreaktoren sind jedoch aufwändig in der Handhabung und sehr teuer. Dies trifft auch für Reaktoren zu, die wie ein Schachtreaktor gebaut sind, aber eine große Anzahl von Rohrschlangen oder Wärmetauscherplatten eingebaut haben, damit genügend Energie zugeführt werden kann.

Aufgabe der vorliegenden Erfindung ist daher die Bereitstellung eines Verfahrens zur Herstellung von delta-Valerolacton mit einer Reinheit > 98% bei dem ein Reaktor verwendet werden kann, bei dem nur die zum Aufheizen der Zufuhrströme benötigte Wärmemenge vor dem Reaktor eingebracht werden muss. Somit wäre der Einsatz auch technisch nicht aufwändiger Reaktoren wie Schachtreaktoren ohne weitere Einbauten möglich. Eine weitere Aufgabe der Erfindung ist es, den Anteil an Nebenkomponenten, insbesondere solchen, die bei Polymerisationsanwendungen zu Kettenabbrüchen führen, oder Komponenten, die die Farbzahlstabilität herabsetzen, wie aldehydische Komponenten, herabzusetzen.

Die Aufgabe wird gelöst durch ein Verfahren zur Herstellung von delta-Valerolacton ausgehend von 1,5-Pentandiol in der Gasphase enthaltend folgende Schritte
a) Verdampfen des 1,5 Pentandiols in einem inerten Gasstrom
b) Durchleiten des 1,5-Pentandiols enthaltenden inerten Gasstroms durch das Katalysatorbett enthaltend wenigstens zwei unterschiedliche Katalysatoren,
c) Auskondensieren von Reaktionsprodukt aus dem aus Schritt b) erhaltenen Gasgemisches,
wobei die eingesetzten Katalysatoren die Elemente und/oder Verbindungen der Elementen der 7. bis 12. Perioden des Periodensystems enthalten und schichtweise so aufgebaut sind, dass der Katalysator, der bei der gegebenen Katalysatorbetteingangstemperatur die höchsten Umsätze und Selektivitäten für die Herstellung von delta-Valerolacton ausgehend von 1,5-Pentandiol zeigt, als erster durchströmt wird und der Katalysator, der bei niedrigeren Temperaturen die höchsten Umsätze und Selektivitäten für die Herstellung von delta-Valerolacton ausgehend von 1,5-Pentandiol zeigt, anschließend durchströmt wird.

Es wurde nun überraschend gefunden, dass man VLO dadurch besonders vorteilhaft herstellen kann, indem man 1,5-Pentandiol in der Gasphase zusammen mit einem inerten Gas über mindestens zwei Katalysatoren leitet, wobei die Katalysatoren bei unterschiedlichen Temperaturen die Reaktion optimal katalysieren.

Bei Einsatz nur eines einzigen Katalysators ist, wie das Vergleichsbeispiel zeigt, entweder die Reaktivität oder die Selektivität nicht optimal, sofern keine weitere Wärmezufuhr während der Reaktion stattfindet. So wird bei Verwendung nur eines Katalysators, der seine höchste Reaktivität und Selektivität bei einer bestimmten Temperatur hat, aufgrund der endothermen Reaktionsenthalpie die Anfangstemperatur so weit abgesenkt, dass Umsatzeinbußen eintreten. Erhöht man dagegen bei einem solchen System, das nur mit einem Katalysator arbeitet, die Anfangstemperatur oberhalb der optimalen Temperatur, so erreicht man zwar einen höheren Umsatz, aber die Selektivität des Katalysators sinkt, was auch zu kurzen Katalysatorstandzeiten führen kann.

Bei dem erfindungsgemäßen Verfahren liegen am Katalysatorbetteingang höhere Temperaturen an als am Katalysatorbettausgang. Bevorzugt liegt die Temperatur am Katalysatorbetteingang im Bereich von 260 bis 350°C, besonders bevorzugt im Bereich von 270 bis 330°C. Am Katalysatorbettausgang liegt die Temperatur bevorzugt im Bereich von 180 bis 240°C, besonders bevorzugt im Bereich von 190 bis 230°C.

Das für die Reaktion eingesetzte Trägergas ist ein inertes Gas. Dieses inerte Trägergas ist bevorzugt ausgewählt aus der Gruppe von Wasserstoff, Stickstoff, Argon und Methan. Besonders bevorzugt ist Wasserstoff. Das molare Verhältnis von 1,5-Pentandiol zu inertem Gas bezogen auf den Reaktoreingang kann zwischen 1 zu 0,1 bis 300 betragen. Bevorzugt ist jedoch die Durchführung mit einem inerten Trägergas im Verhältnis 1,5-Pentandiol zu inertem Gas von 1 zu 0,5 bis 200, besonders bevorzugt von 1 zu 2 bis 150.

Der Druck wird bei den Schritten a) bis c) des erfindungsgemäßen Verfahrens bevorzugt so eingestellt, dass sich in dem Reaktor keine Flüssigphase bildet. Besonders bevorzugt liegt der Druck zwischen 0,5 und 10 bar absolut, ganz besonders bevorzugt zwischen 1 bis 5 bar absolut.

Die Katalysatoren, die im erfindungsgemäßen Verfahren eingesetzt werden sind solche, die Elemente und/oder Verbindungen der Elemente der 7. bis 12. Gruppe des Periodensystems enthalten. Die erfindungsgemäßen Katalysatoren können oxidische Katalysatoren enthalten, bevorzugt sind solche, die üblicherweise zu Dehydrierreaktionen eingesetzt werden, beispielsweise der Dehydrierung von Cyclohexanol zu Cyclohexanon. Diese Übergangsmetallelemente der 7. bis 12. Gruppe des Periodensystems können elementar oder zumindest zum Teil oxidisch vorliegen. Bevorzugte Übergangsmetallelemente sind ausgewählt aus der Gruppe von Zn, Ag, Cu, Ru und Au, besonders bevorzugt ist Cu. Es können zum Zwecke der Feinsteuerung der Katalysatoreigenschaften noch weitere Elemente als Dotierungen enthalten sein. Diese Dotierungen sind beispielsweise basische Komponenten, wie Oxide, Hydroxide oder Carboxylate der Elemente der Gruppen 1-14. Bevorzugt sind basische Komponenten der Alkali- und Erdalkalielemente.

Die Übergangsmetallelemente sind auf oxidischen Trägermaterialen oder Aktivkohlen aufgebracht. Bevorzugte Trägermaterialen sind Aktivkohlen und Oxide von Al, Si, Zn, Ti, Fe, Cr, Zr. Es können auch Mischungen der einzelnen Oxide oder Mischverbindungen wie sie in Zeolithen und Tonerden vorkommen.

Die Gehalte der Übergangsmetallelemente auf den Trägern liegen zwischen 3 und 97 Gew.-%, bevorzugt sind 5 bis 80 Gew.-%, besonders bevorzugt sind 10 bis 70 Gew.-% Die Katalysatoren können vor Einbau in den Reaktor aktiviert werden aber auch in situ im Reaktor. Werden die Katalysatoren vor dem Einbau aktiviert, so ist es vorteilhaft, wenn diese nach Aktivierung passiviert werden, vor allem wenn die Aktivkomponente metallisch vorliegen soll. Es ist auch möglich, die aktivierten Katalysatoren ohne Passivierung, dafür in einem inerten Lösemittel wie Pentandiol zum Einbau zu verwenden.

In dem erfindungsgemäßen Verfahren wird das delta-Valerolacton durch Umsetzung von 1,5-Pentandiol an mindestens zwei hintereinander geschaltenen Katalysatorschichten hergestellt. Für das erfindungsgemäße Verfahren können auch noch weitere Schichten anderer Katalysatoren vor, zwischen oder hinter den beiden Schichten eingefügt werden. Voraussetzung für ein zwei wie auch für ein mehr als zwei Schichten enthaltendes Katalysatorbett ist jedoch, dass die Schichten im Katalysatorbett so aufgebaut sind, dass der Katalysator, der bei der höchsten Temperatur die höchsten Umsätze und Selektivitäten für die Umsetzung von 1,5-Pentandiol zu delta-Valerolacton zeigt, als erster durchströmt wird und alle weiteren Katalysatorschichten folgen, die bei stetig fallenden Temperaturen die höchsten Umsätze und Selektivitäten für die erfindungsgemäße Umsetzung zeigen. Für das erfindungsgemäße Verfahren ist es dabei unerlässlich, dass das Katalysatorbett, das mindestens zwei unterschiedliche Katalysatoren enthält, in Schichten aufgebaut ist, die sich nicht durchmischen.

Im erfindungsgemäßen Schritt c) wird der Gasstrom aus dem Reaktor so abgekühlt, dass die unter Normalbedingungen flüssigen Reaktionskomponenten zumindest weitgehend auskondensiert werden. Dies kann durch klassische Wärmetauscher geschehen, prinzipiell ist jedoch auch oder zusätzlich ein Auswaschen dieser Komponenten mit einem bereits flüssigen Stoff möglich. Bevorzugt wird zum Auswaschen das flüssige Reaktionsprodukt selbst verwendet.

Nach dem erfindungsgemäßen Schritt c) kann in einem weiteren Schritt d) ein Teil des inerten Gases, z.B. der während der Reaktion entstehende aus dem Verfahren ausgeschleust werden. Der Rest kann dem erfindungsgemäßen Verfahren in Schritt a) wieder zugeführt werden. Bevorzugt wird dies durch ein Kreisgasgebläse realisiert.

Die so gewonnen flüssigen Bestandteile des Schrittes c) können in einem weiteren Schritt e) einer destillativen oder rektikativen Aufarbeitung zugeführt. Dabei kann gegebenenfalls enthaltenes 1,5-Pentandiol nach Abtrennung in Schritt a) des erfindungsgemäßen Verfahrens rückgeführt und reines Valerolacton erhalten werden. Die Reinheiten des Valerolactons liegen dabei über 98 %, bevorzugt über 99 %.

### Beispiele:

Das erfindungsgemäße Verfahren wird mittels der nachstehenden Beispiele näher erläutert, jedoch nicht eingeschränkt. Die in den Beispielen aufgeführten gaschromatographischen (GC) %-Angaben sind Flächen-%.

### Beispiel 1 (erfindungsgemäß):

Ein Rohrreaktor mit einem Innendurchmesser von 32 mm und einem außenliegenden Mantel, der dazu da ist, Wärmeverluste aus dem Reaktor zu vermeiden, wurde in einer ersten Schicht mit 25 ml eines Kupferkatalysators (Katalysator 1, 5x3 mm Tabletten, 21 % Kupferoxid, 2 % Natriumoxid, Rest SiO2, hergestellt durch Tränkung von ammoniakalischer Kupfercarbonat/Natriumnitrat-Lösung auf SiO2-Strängen, gefolgt durch Trocknung und Kalzinierung) und mit einer zweiten Schicht darunter mit 25 ml eines zweiten Kupferkatalysators (Katalysator 2, 5 mm Stränge, 13 % Kupferoxid, 1 % Natriumoxid, 7 % CaO, Rest SiO2, hergestellt durch Tränkung von ammoniakalischer Kupfercarbonat/Natriumnitrat-Lösung auf CaO/SiO2-Strängen, gefolgt durch Trocknung und Kalzinierung) befüllt. Oberhalb des Rohrreaktors befand sich eine Heizzone, die als Verdampfer benutzt wurde und mittels derer die Reaktoreingangstemperatur variiert werden konnte.

Mittels eines Stickstoff/Wasserstoffgemischs von 10 zu 1 (10 Normliter/h) wurden die Katalysatoren bis zu 180°C innerhalb von 72 h aktiviert.

Mittels einer Pumpe wurden danach 1,5-Pentandiol (Reinheit 97 %, ca. 4 g/h) und Wasserstoff 10 Normliter/h) bei Normaldruck über den Verdampfer zudosiert. Die Temperatur am Reaktoreingang wurde auf 300°C eingestellt. Eine Temperaturmessung zu Beginn der zweiten Katalysatorschicht stellte sich auf ca. 260°C ein. Der Austrag wurde auf ca. 10°C abgekühlt, die anfallende Flüssigkeit wurde aufgefangen, das Gas wurde ausgeschleust. Die Reaktion wurde über einen Zeitraum von 700 h betrieben, dann abgebrochen. Nach 24 h und auch noch nach 700 h lag der 1,5-Pentandiol-Umsatz über 99 %. Der Gehalt an VLO im flüssigen Austrag lag über diesen Zeitraum zwischen 93 und 94 %, das Zwischenprodukt 5-Hydroxypentanal lag immer deutlich unter 0,2 %. Die Valerolactonselektivität lag demnach zwischen 96 und 97 %.

Die flüssigen Reaktionsausträge wurden z.T. aufdestilliert. Es konnten VLO-Reinheiten von über 99% erreicht werden.

### Beispiel 2 (Vergleichsbeispiel)

Beispiel 1 wurde wiederholt, mit dem Unterschied, dass anstatt Katalysator 2 zusätzlich 25 ml Katalysator 1 eingefüllt wurden.

Der 1,5-Pentandiol-Umsatz lag innerhalb der ersten 300 h bei knapp 99 %, der Gehalt am Zwischenprodukt 5-Hydroxypentanal betrug bis zu 4 %, entsprechend lag der VLO-Gehalt zwischen 90 und 92 %. Nach 300 h fiel der Pentandiol-Umsatz laufend ab und erreichte nach 580 h nur noch ca. 94 %. Die VLO-Selektivitäten erreichten maximal 95 %. Nach 580 h wurde der Versuch abgebrochen.

Die Destillation der Austräge ergab VLO-Reinheiten bis zu 98 %, wobei jedoch untolerierbar hohe Anteile an 5-Hydroxypentanal enthalten waren, so dass das erhaltene Produkt nicht farbzahlstabil war.

### Beispiel 3 (Vergleichsbeispiel)

Beispiel 1 wurde wiederholt, mit dem Unterschied, dass anstatt Katalysator 1 25 ml an Katalysator 2 zusätzlich eingefüllt wurden.

Der Umsatz an 1,5-Pentandiol war zu Beginn zwar über 99 %, allerdings lag die VLO-Selektivität unter 90 % und die Zunahme an 5-Hydroxypentanal sowie anderer Produkten stieg so stark an, dass der Versuch bereits nach 100 h abgebrochen werden musste.

### Beispiel 4 (Vergleichsbeispiel)

Beispiel 3 wurde wiederholt, mit dem Unterschied, dass die Reaktoreingangstemperatur bei 260°C lag. Der Umsatz an 1,5-Pentandiol betrug nur ca. 85 %, so dass dieser Versuch ebenfalls nach 100 h abgebrochen wurde.

Der Katalysator des Vergleichsbeispieles 4 besitzt eine optimale Reaktivität bei optimaler Selektivität zwischen 230 und 250°C. Bringt man nun einen gasförmigen Stoffstrom bestehend aus 1,5-Pentandiol und Wasserstoff in einem Verhältnis zueinander ein, das bis zum Erreichen eines maximalen Umsatzes 50°C Temperaturabkühlung aufgrund der endothermen Reaktionsenthalpie bedingte, erhält man bei einer Reaktoreingangstempertur von 250°C und einer Reaktoraustrittstemperatur von 220 - 230°C nur ca. 50 % des möglichen Umsatzes. Bei erhöhter Eingangstemperatur, beispielsweise 280°C, konnte man zwar ausreichend Umsatz erreichen, allerdings bei verminderter Selektivität und auch unzureichender Katalysatorstandzeit.

## Patentansprüche

1. Verfahren zur Herstellung von delta-Valerolacton ausgehend von 1,5 Pentandiol enthaltend folgende Schritte
a) Verdampfen des 1,5-Pentandiols in einem inerten Gasstrom
b) Durchleiten des 1,5-Pentandiol enthaltenden inerten Gasstromes durch das Katalysatorbett enthaltend wenigstens zwei unterschiedliche Katalysatoren
c) Auskondensieren von Reaktionsprodukt aus dem aus Schritt b) erhaltenen Gasgemisches,
wobei die eingesetzten Katalysatoren die Elemente und/oder Verbindungen der Elementen der 7. bis 12. Perioden des Periodensystems enthalten und schichtweise so aufgebaut sind, dass der Katalysator, der bei der gegebenen Katalysatorbetteingangstemperatur die höchsten Umsätze und Selektivitäten für die Herstellung von delta-Valerolacton ausgehend von 1,5-Pentandiol zeigt, als erster durchströmt wird und der Katalysator, der bei niedrigeren Temperaturen die höchsten Umsätze und Selektivitäten für die Herstellung von delta-Valerolacton ausgehend von 1,5-Pentandiol zeigt, anschließend durchströmt wird.

2. Verfahren nach Anspruch 1, wobei das eingesetzte inerte Gas ausgewählt ist aus der Gruppe von Wasserstoff, Stickstoff, Argon und Methan.

3. Verfahren nach einem der Ansprüche 1 bis 2, wobei das Verhältnis von1,5-Pentandiol zu inertem Gas im Bereich von 1 zu 0,1 bis 300 liegt.

4. Verfahren nach einem der Ansprüche 1 bis 3, wobei das eingesetzte inerte Gas Wasserstoff ist und das Verhältnis von 1,5-Pentandiol zu Wasserstoff im Bereich von 1 zu 2 bis 150 liegt.

5. Verfahren nach einem der Ansprüche 1 bis 4, wobei die eingesetzten Katalysatoren Elemente und/oder Verbindungen der Übergangsmetalle ausgewählt aus der Gruppe von Kupfer, Ruthenium und Gold enthalten.

6. Verfahren nach einem der Ansprüche 1 bis 5, wobei die eingesetzten Katalysatoren zusätzlich noch Alkalioxide enthalten.

7. Verfahren nach einem der Ansprüche 1 bis 6, wobei die eingesetzten Katalysatoren auf Trägermaterialien ausgewählt aus der Gruppe von Aktivkohle, Oxiden ausgewählt aus der Gruppe von Aluminium, Silizium, Zink, Titan, Eisen, Chrom und Zirkonium und Mischungen dieser Oxide aufgebracht sind.

8. Verfahren nach einem der Ansprüche 1 bis 7, wobei vor, zwischen und/oder hinter den beiden im Katalysatorbett wenigstens enthaltenen Katalysatorschichten noch weitere Katalysatorschichten enthalten sein können, die so aufgebaut sind, dass die Katalysatorschicht, die bei der Katalysatorbetteingangstemperatur den höchsten Umsatz und die höchste Selektivität für die Umsetzung von 1,5-Pentandiol zu delta-Valerolacton zeigt als erste durchströmt wird und anschließend die Katalysatorschichten, die bei stetig fallender Temperatur die höchsten Umsätze und Selektivitäten für die Umsetzung von 1,5-Pentandiol zu delta-Valerolacton zeigen, entsprechend ihrer Umsatz- und Selektivitätsrate folgen.

9. Verfahren nach einem der Ansprüche 1 bis 8, wobei das Verfahren bei einem Druck im Bereich von 1 bis 5 bar absolut liegt.

10. Verfahren nach einem der Ansprüche 1 bis 9, wobei ein Teil des aus Schritt c) erhaltenen inerten Gases in Schritt a) des Verfahrens zurückgeführt wird.

11. Verfahren nach einem der Ansprüche 1 bis 10, wobei die aus Schritt c) erhaltenen flüssigen Reaktionskomponenten einer Destillation oder Rektifikation unterzogen werden.

12. Verfahren nach Ansprüche 1 bis 11, wobei das nach der Destillation oder Rektifikation gewonnene nicht umgesetzte 1,5-Pentandiol in Schritt a) des Verfahrens zurückgeführt wird.

## Claims

1. A process for preparing delta-valerolactone proceeding from 1,5 pentanediol, comprising the following steps:
a) evaporating the 1,5-pentanediol in an inert gas stream
b) passing the inert gas stream comprising 1,5-pentanediol through the catalyst bed comprising at least two different catalysts
c) condensing reaction product out of the gas mixture obtained from step b),
wherein the catalysts used comprise the elements and/or compounds of the elements of periods 7 to 12 of the Periodic Table and have a layer structure such that the inert gas stream comprising 1,5-pentanediol flows first through the catalyst which exhibits the highest conversions and selectivities for the preparation of delta-valerolactone proceeding from 1,5-pentanediol at the given catalyst bed inlet temperature, and then through the catalyst which exhibits the highest conversions and selectivities for the preparation of delta-valerolactone proceeding from 1,5-pentanediol at lower temperatures.

2. The process according to claim 1, wherein the inert gas used is selected from the group of hydrogen, nitrogen, argon and methane.

3. The process according to either of claims 1 and 2, wherein the ratio of 1,5-pentanediol to inert gas is in the range from 1:0.1 to 300.

4. The process according to any one of claims 1 to 3, wherein the inert gas used is hydrogen and the ratio of 1,5-pentanediol to hydrogen is in the range from 1:2 to 150.

5. The process according to any one of claims 1 to 4, wherein the catalysts used comprise elements and/or compounds of the transition metals selected from the group of copper, ruthenium and gold.

6. The process according to any one of claims 1 to 5, wherein the catalysts used additionally also comprise alkali metal oxides.

7. The process according to any one of claims 1 to 6, wherein the catalysts used are applied to support materials selected from the group of activated carbon, oxides selected from the group of aluminum, silicon, zinc, titanium, iron, chromium and zirconium, and mixtures of these oxides.

8. The process according to any one of claims 1 to 7, wherein further catalyst layers may be present upstream of, between and/or downstream of the two catalyst layers at least present in the catalyst bed, and have a structure such that the inert gas stream comprising 1,5-pentanediol flows first through the catalyst layer which exhibits the highest conversion and the highest selectivity for the conversion of 1,5-pentanediol to delta-valerolactone at the catalyst bed inlet temperature, followed by the catalyst layers which exhibit the highest conversions and selectivities for the conversion of 1,5-pentanediol to delta-valerolactone at constantly falling temperature, according to their conversion and selectivity rates.

9. The process according to any one of claims 1 to 8, which is at a pressure in the range from 1 to 5 bar absolute.

10. The process according to any one of claims 1 to 9, wherein a portion of the inert gas obtained from step c) is recycled to step a) of the process.

11. The process according to any one of claims 1 to 10, wherein the liquid reaction components obtained from step c) are subjected to a distillation or rectification.

12. The process according to claims 1 to 11, wherein the unconverted 1,5-pentanediol obtained after the distillation or rectification is recycled to step a) of the process.

## Revendications

1. Procédé de préparation de delta-valérolactone à partir de 1,5-pentanediol, comprenant les étapes suivantes :
a) l'évaporation du 1,5-pentanediol dans un courant gazeux inerte,
b) le passage du courant gazeux inerte contenant le 1,5-pentanediol au travers du lit catalytique contenant au moins deux catalyseurs différents,
c) la condensation du produit de réaction à partir du mélange gazeux obtenu à l'étape b),
les catalyseurs utilisés contenant les éléments et/ou des composés des éléments des périodes 7 à 12 du tableau périodique et étant formés en couches de manière à ce que le catalyseur qui présente à la température d'entrée dans le lit catalytique donnée les conversions et sélectivités les plus élevées pour la préparation de delta-valérolactone à partir de 1,5-pentanediol soit traversé en premier et à ce que le catalyseur qui présente à des températures plus basses les conversions et sélectivités les plus élevées pour la préparation de delta-valérolactone à partir de 1,5-pentanediol soit traversé ensuite.

2. Procédé selon la revendication 1, dans lequel le gaz inerte utilisé est choisi dans le groupe constitué par l'hydrogène, l'azote, l'argon et le méthane.

3. Procédé selon l'une quelconque des revendications 1 à 2, dans lequel le rapport entre le 1,5-pentanediol et le gaz inerte se situe dans la plage allant de 1 sur 0,1 à 300.

4. Procédé selon l'une quelconque des revendications 1 à 3, dans lequel le gaz inerte utilisé est l'hydrogène et le rapport entre le 1,5-pentanediol et l'hydrogène se situe dans la plage allant de 1 sur 2 à 150.

5. Procédé selon l'une quelconque des revendications 1 à 4, dans lequel les catalyseurs utilisés contiennent des éléments et/ou des composés des métaux de transition choisis dans le groupe constitué par le cuivre, le ruthénium et l'or.

6. Procédé selon l'une quelconque des revendications 1 à 5, dans lequel les catalyseurs utilisés contiennent également des oxydes alcalins.

7. Procédé selon l'une quelconque des revendications 1 à 6, dans lequel les catalyseurs utilisés sont appliqués sur des matériaux supports choisis dans le groupe constitué par le charbon actif, les oxydes choisis dans le groupe constitué par l'aluminium, le silicium, le zinc, le titane, le fer, le chrome et le zirconium et les mélanges de ces oxydes.

8. Procédé selon l'une quelconque des revendications 1 à 7, dans lequel, avant, entre et/ou après les deux couches de catalyseur ou plus contenues dans le lit catalytique, d'autres couches de catalyseur peuvent encore être contenues, qui sont formées de manière à ce que la couche de catalyseur qui présente à la température d'entrée dans le lit catalytique la conversion la plus élevée et la sélectivité la plus élevée pour la transformation de 1,5-pentanediol en delta-valérolactone soit traversée en premier, puis les couches de catalyseur qui présentent à une température diminuant continuellement les conversions et sélectivités les plus élevées pour la transformation de 1,5-pentanediol en delta-valérolactone suivent en fonction de leurs taux de conversion et de sélectivité.

9. Procédé selon l'une quelconque des revendications 1 à 8, dans lequel le procédé est réalisé à une pression dans la plage allant de 1 à 5 bar absolu.

10. Procédé selon l'une quelconque des revendications 1 à 9, dans lequel une partie du gaz inerte obtenu à l'étape c) est recyclée dans l'étape a) du procédé.

11. Procédé selon l'une quelconque des revendications 1 à 10, dans lequel les composants réactionnels liquides obtenus à l'étape c) sont soumis à une distillation ou à une rectification.

12. Procédé selon les revendications 1 à 11, dans lequel, le 1,5-pentanediol non réagi obtenu après la distillation ou la rectification est recyclé dans l'étape a) du procédé.
